# EUROPEAN PATENT SPECIFICATION

(11) **EP 3 294 778 B1**
(45) Date of publication and mention of the grant of the patent: **10.04.2019**
(21) Application number: 16733689.0
(22) Date of filing: 13.05.2016
(51) Int. Cl.: C08B 37/08, A61K 31/728

(54) **HYALURONIC ACID MICRO-SPONGES AND METHOD FOR THE PRODUCTION THEREOF**
MIKROSCHWÄMME AUS HYALURONSÄURE UND METHODEN ZU DEREN HERSTELLUNG
MICRO-ÉPONGES DE ACIDE HYALURONIQUE ET PROCEDE DE PRODUCTION

(30) Priority: 13.05.2015 IT RM20150204
(43) Date of publication of application: 21.03.2018
(73) Proprietor: Nanofaber S.r.L., 00154 Roma (IT)
(72) Inventor: CAVALIERI, Francesca, 00154 Roma (IT)
(74) Representative: Bosman, Cesare
(86) International application number: PCT/IB2016/052792
(87) International publication number: WO 2016/181365

(56) References cited:
- EP-A1- 2 088 140
- US-A1- 2007 224 277
- LEE ET AL: "Target-specific intracellular delivery of siRNA using degradable hyaluronic acid nanogels", JOURNAL OF CONTROLLED RELEASE, ELSEVIER, AMSTERDAM, NL, vol. 119, no. 2, 16 May 2007 (2007-05-16), pages 245-252, XP022080065, ISSN: 0168-3659, DOI: 10.1016/J.JCONREL.2007.02.011

## Description

### TECHNICAL FIELD

The present invention relates to micro-sponges made of hyaluronic acid and a method for the production thereof.

### PRIOR STATE OF THE ART

Hyaluronic acid is an unbranched, linear polysaccharide composed of repeating disaccharides units consisting of glucuronic acid and N-acetylglucosamine,and it is one of the fundamental constituents of the human connective tissues. In particular, hyaluronic acid is found in the vitreous humor of the eye, in the synovial fluid, in the skin, in the cartilage, in tendons, in the umbilical cord, and in the aorta walls.

The use of hyaluronic acid for medical application has been known for a long time. In fact, the hyaluronic acid is used in surgery and aesthetic dermatology to eliminate wrinkles and prevent aging of the skin; in otologic surgery as regenerator of perforated tympanic membranes; in ophthalmic surgery for the production of artificial tearsin cataract and retina surgery, intraocular lens implantation, retinal detachment surgery, corneal transplant surgery, vitrectomy and traumatic eye surgery; and in arthrology as an antiphlogistic lubricant and preservative of the synovial fluid of the joints. In addition, hyaluronic acid is also employed against inflammation and ulcerative lesions of the mouth.

One of the problems encountered in the application of hyaluronic acid is its fast degradation. This problem implies that the therapeutic effects of hyaluronic acid have a short life span from the time of its administration. Moreover, the application of hyaluronic acid is very often invasive in nature and, therefore, cannot be repeated with a high frequency.

There was therefore the need to apply the hyaluronic acid in a form such to prevent its rapid degradation and, thus, to ensure a longer life span of its medical effects after a single administration.

The inventors of the present invention have surprisingly found that the reaction between a cross-linking agent and a hyaluronic acid, having a specific range of average molecular weight, yields uniform hyaluronic acid micro-sponges with a degradation rate far lower than the hyaluronic acid commonly used in the medical field. As it is apparent to the skilled person in this field, a lower rate of degradation necessarily results into greater retention, as verifiable from in vivo experiments.

### OBJECT OF THE INVENTION

Object of the present invention is a method for the preparation of hyaluronic acid micro-sponges, whose essential characteristics are reported in claim 1, and whose preferred characteristics and / or auxiliaries are set forth in claims 2-4.

A further object of the present invention consists of hyaluronic acid micro-sponges, the essential characteristics of which are reported in claim 5.

### BRIEF DESCRIPTION OF DRAWINGS

For a better understanding of the invention, a real example - yet purely illustrative and non-limiting - is provided in the following with the aid of accompanying figures (1A, 1B, 1C, 1D), which are images of the micro-sponges object of the present invention taken at different resolution.

### PREFERRED FORM OF REALIZATION OF THE INVENTION

### Preparation of cross-linking agent:

30 mg of 1,1'-carbonyldiimidazole (CDI) and 20 mg of cystamine were dissolved in 200 µl of anhydrous DMSO and the resulting solution was kept under stirring for 5 hours at room temperature. At this stage, the CDI reacts with cystamine to achieve the cross-linking agent. In particular, the cystamine reacts with CDI through its amino groups.

Alternatively, 30 mg of 1,1'-carbonyldiimidazole (CDI) and 20 mg of cystamine were dissolved in 200 µl of water (Milli-Q), and the pH of the solution was brought to 4-5 for solubilizing the cross-linking agent.

### Cross-linking/precipitation of hyaluronic acid:

An aqueous solution of 1 g/dL of hyaluronic acid with an average MW of 800 kDa was prepared. The solution was kept under stirring overnight.

The average molecular weights given in the description are calculated by the method in the international standard ISO 11344.

The solution of the cross-linking agent described above was added to 0.5 ml of the solution of the aqueous hyaluronic acid. The resulting solution was kept under stirring for a whole night at room temperature to favor the reaction between the hyaluronic acid and the cross-linking agent. Such a reaction led to the cross-linking of hyaluronic acid and to the precipitation of micro-sponges of substantially spherical and porous shape. The reaction between the cross-linking agent and the hyaluronic acid involves the hydroxylic and carboxylic groups of the latter one.

In addition to react with the cross-linking agent through its hydroxylic and carboxylic groups, the hyaluronic acid also plays a nucleation function for the formation of nano crystals of cross-linking agent. In fact, it was verified that the presence of hyaluronic acid makes it possible to control the size and homogeneity of the crystals of cross-linking agent, which otherwise would be large and irregular.

It was verified that CO₂ is produced during the cross-linking reaction, which acts as a porogen agent.

The micro-sponges were harvested by centrifugation and washed several times in distilled water to be then preserved in an aqueous suspension at 4°C.

The morphological characterization of the micro-sponges was conducted by the SEM technique. A high-resolution SEM microscope (FEG-SEM, LEO 35) was used at low-voltage 0.5 - 2.0 kV to limit the damage on the micro-sponges.

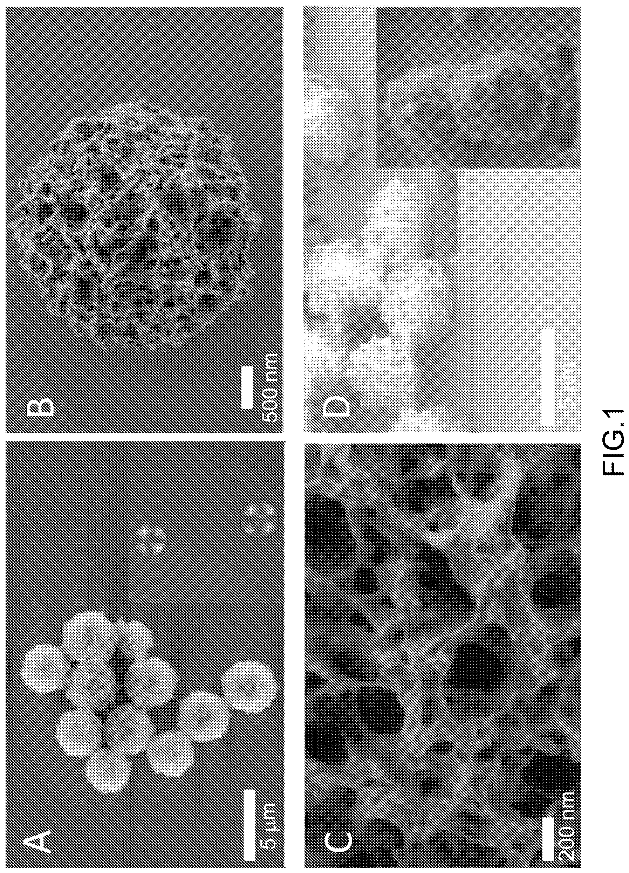

In order to study their outer and inner structure, the micro-sponges were observed in a scanning electron microscope (SEM) and sectioned using a dual-beam FIB (EIF-Helios Nanolab 600) with a source of liquid metal ions of gallium. These images are displayed in the Figures 1A-1D attached here.

The hyaluronic acid micro-sponges produced in this manner were measured to have a spherical shape with an average diameter of about 4.2 µm. In particular, it was found that the hyaluronic acid micro-sponges had an average diameter of 3.3 ± 0.9 µm in the absence of water and an average diameter of 4.2 ± 0.9 µm when swollen due to the presence of water. Furthermore, the hyaluronic acid micro-sponges made as described above had a mean degree of crosslinking of 0.0072 mol/cm³, an average density of 1.04 g/cm³ and a pore size between 130 and 270 nm.

### Degradation tests:

The hyaluronic acid micro-sponges produced with the method described above were immersed in an environment suitable to simulate the degradation conditions, and their degradation time was observed.

The degradative conditions were carried out with a solution of hyaluronidase (0.5 mg/mL) in phosphate buffered saline (PBS) at pH 7. Under the above conditions a 10% degradation of the micro-sponges was observed in the first 24h and a degradation of only 20% was observed over the next nine days. For proper appreciation of these results, it has to be borne in mind that the hyaluronic acid in the commonly used form is completely degraded after just eight hours in the said conditions.

The above results demonstrate how the structure of the micro-sponges of hyaluronic acid from the present invention is able to prevent the enzymatic degradation.

The inventors found that, in order to ensure a low degradation, the hyaluronic acid micro-sponges have to have a pore size ranging from 10 to 600 nm, preferably between 100 and 300 nm, and a degree of cross-linking ranging from 0.001 to 0.01 mol/cm³, preferably between 0.005 and 0.008 mol/cm³.

To realize the present invention, the molecular weight of hyaluronic acid has to range from 250,000 to 1,500,000 Da, preferably between 500,000 and 1000,000 Da, while the concentrations of the cross-linking agent and of the hyaluronic acid have to range respectively from 0.05 to 0.15 M (preferably between 0.05 and 0.1 M) and from 0.5 to 1.5 g/dL (preferably between 0.8 and 1.0 g/dL).

It was experimentally verified that the use of hyaluronic acid with a molecular weight lower than that indicated above fails to achieve a uniform spherical structure and requires longer reaction times, whereas the use of hyaluronic acid with a molecular weight greater than indicated above leads to an increase in the viscosity of the hyaluronic acid solution, with resulting inhomogeneity of the reactive mixture.

It was experimentally found that, if the hyaluronic acid has a concentration lower than 0.5 g/dL, the resulting micro-sponges are not uniform and polydispersed, whereas, if the hyaluronic acid has a concentration greater than 5 g/dL, the resulting solution is too viscous, thus preventing that the method of the present invention is correctly realized.

Preferably, the cross-linking agent comprises a chain with "cleavable" groups, for example by means of hydrolysis or redox reactions. In this way, it will be possible to incorporate a substance within the hyaluronic acid micro-sponges, and then cause its release into a physiological environment through the degradation of these cleavable groups, with consequent solubilization of the micro-sponges. The substances that may be incorporated within the micro-sponges object of the present invention are related to molecules, macromolecules, and nano particles with a positive charge.

At last, preferably the hyaluronic acid micro-sponges of the present invention have a cavity of size ranging from 1 to 3 microns, and even more preferably between 0.8 and 2 microns.

The hyaluronic acid micro-sponges of the present invention offer the great advantage of having a degradation rate by far lower than that of the hyaluronic acid commonly used in medical applications. This advantage results in a prolonged medical effect of the hyaluronic acid upon its administration.

The hyaluronic acid micro-sponges of the present invention find a preferred and advantageous deployment in all those applications where the hyaluronic acid is used on the human body, particularly in those cases where it is required that the hyaluronic acid be injected into the human or animal body. In fact, the slow degradation of the hyaluronic acid in these cases averts the need for frequent injections of it. These applications pertain particularly the fields of cosmetic surgery and arthrology.

## Claims

1. A method to produce hyaluronic acid micro-sponges **characterized in that** it comprises a concomitant cross-linking and precipitation step, during which the hyaluronic acid at a concentration raging from 0.5 to 1.5 g/dL reacts with the cross-linking agent in a concentration ranging from 0.05 to 0.15 M; said hyaluronic acid having a molecular weight ranging from 250,000 to 1500,000; said cross-linking agent being prepared by causing 1,1'-carbonyldiimidazole (CDI) to react with a diamine compound.

2. A method according to claim 1, **characterized in that** the hyaluronic acid has an average molecular weight ranging from 500,000 to 1000,000 Da, and **in that**, during said cross-linking step, the concentration of the hyaluronic acid ranges from 0.8 to 1.0 g/dL, and the concentration of cross-linking agent ranges from 0.05 to 0.10 M.

3. A method according to claim 1 or 2, **characterized in that** said cross-linking agent comprises chains with cleavable functional groups.

4. A method according to claim 1, **characterized in that** said diamine compound has a chain comprising cleavable functional groups.

5. Hyaluronic acid micro-sponges prepared by the methods of claims 1-4, **characterized in that** they have an average diameter ranging from 1.5 to 10.0 µm, the average size of pores ranging from 10 to 400 nm, and a cross-linking degree ranging from 0.001 to 0.01 mol/cm³.

6. Hyaluronic acid micro-sponges according to claim 5, **characterized in that** they have an average diameter ranging from 4.0 to 8.0 µm, an average pore size ranging from 100 to 300 nm, and a cross-linking degree ranging from 0.005 to 0.008 mol/cm³.

7. Hyaluronic acid micro-sponges according to claim 5 or 6, **characterized in that** they incorporate, on the inside, molecules, macromolecules and nano particles with a positive charge.

8. Hyaluronic acid micro-sponges according to one of claims 5 to 7, for use in medical or cosmetic applications.

9. A compound for medical or cosmetic purposes, **characterized in that** it comprises hyaluronic acid micro-sponges according to any of claims from 5 to 7.

## Patentansprüche

1. Verfahren zur Herstellung von Hyaluronsäure-enthaltenden Mikroschwämmen,
**dadurch gekennzeichnet, dass** es umfasst einen gleichzeitigen Vernetzungs- und Präzipitationsschritt, während dem die Hyaluronsäure bei einer Konzentration im Bereich von 0,5 bis 1,5 g/dL mit dem Vernetzungsmittel in einer Konzentration im Bereich von 0,05 bis 0,15 M umgesetzt wird; wobei die Hyaluronsäure ein Molekulargewicht im Bereich von 250.000 bis 1.500.000 besitzt, wobei das Vernetzungsmittel durch Umsetzung von 1,1'-Carbonyldiimidazol (CDI) mit einer Diaminverbindung umgesetzt wird.

2. Verfahren nach Anspruch 1,
**dadurch gekennzeichnet, dass** die Hyaluronsäure ein mittleres Molekulargewicht im Bereich von 500.000 bis 1.000.000 Da aufweist, und dadurch, dass während des Vernetzungsschrittes die Konzentration der Hyaluronsäure im Bereich von 0,8 bis 1.0 g/dL liegt, und die Konzentration des Vernetzungsmittels im Bereich von 0,05 bis 0,10 M liegt.

3. Verfahren nach Anspruch 1 oder 2,
**dadurch gekennzeichnet, dass** das Vernetzungsmittel abspaltbare funktionelle Gruppen aufweist.

4. Verfahren nach Anspruch 1,
**dadurch gekennzeichnet, dass** die Diaminoverbindung eine Kette aufweist, welche abspaltbare funktionelle Gruppen aufweist.

5. Hyaluronsäure enthaltende Mikroschwämme, hergestellt durch die Verfahren nach den Ansprüchen 1-4,
**dadurch gekennzeichnet, dass** sie einen mittleren Durchmesser im Bereich von 1,5 bis 10,0 µm aufweisen, die mittlere Porengröße im Bereich von 10 bis 400 nm liegt, und sie einen Vernetzungsgrad im Bereich von 0,001 bis 0,01 mol/cm³ besitzen.

6. Hyaluronsäure enthaltende Mikroschwämme nach Anspruch 5,
**dadurch gekennzeichnet, dass** sie ein mittleren Durchmesse im Bereich von 4,0 bis 8,0 µm, eine mittlere Porengröße im Bereich von 100 bis 300 nm, und einen Vernetzungsgrad im Bereich von 0,005 bis 0,008 mol/cm³ aufweisen.

7. Hyaluronsäure enthaltende Mikroschwämme nach Anspruch 5 oder 6, **dadurch gekennzeichnet, dass** sie, auf der Innenseite, Moleküle, Makromoleküle und Nanoteilchen mit positiver Ladung beinhalten.

8. Hyaluronsäure enthaltende Mikroschwämme nach einem der Ansprüche 5 bis 7 zur Verwendung bei medizinischen oder kosmetischen Anwendungen.

9. Eine Verbindung für medizinische oder kosmetische Zwecke,
**dadurch gekennzeichnet, dass** sie Hyaluronsäure enthaltende Mikroschwämme nach irgendeinem der Ansprüche 5 bis 7 umfasst.

## Revendications

1. Procédé pour produire des micro-éponges d'acide hyaluronique, **caractérisé en ce qu'**il comprend une étape de réticulation et de précipitation concomitantes, durant laquelle l'acide hyaluronique, à une concentration située dans la plage allant de 0,5 à 1,5 g/dl, réagit avec l'agent de réticulation à une concentration située dans la plage allant de 0,05 à 0,15 M ; ledit acide hyaluronique ayant une masse moléculaire située dans la plage allant de 250 000 à 1 500 000 ; ledit agent de réticulation étant préparé par l'opération consistant à faire réagir du 1,1'-carbonyl-diimidazole (CDI) avec un composé diamine.

2. Procédé selon la revendication 1, **caractérisé en ce que** l'acide hyaluronique a une masse moléculaire moyenne située dans la plage allant de 500 000 à 1 000 000 Da, et **en ce que**, durant ladite étape de réticulation, la concentration de l'acide hyaluronique est située dans la plage allant de 0,8 à 1,0 g/dl, et la concentration de l'agent de réticulation est située dans la plage allant de 0,05 à 0,10 M.

3. Procédé selon la revendication 1 ou 2, **caractérisé en ce que** ledit agent de réticulation comprend des chaînes ayant des groupes fonctionnels pouvant être clivés.

4. Procédé selon la revendication 1, **caractérisé en ce que** ledit composé diamine a une chaîne comprenant des groupes fonctionnels pouvant être clivés.

5. Micro-éponges d'acide hyaluronique préparées par les procédés des revendications 1 à 4, **caractérisées en ce qu'**elles ont un diamètre moyen situé dans la plage allant de 1,5 à 10,0 µm, une taille moyenne des pores située dans la plage allant de 10 à 400 nm, et un degré de réticulation situé dans la plage allant de 0,001 à 0,01 mol/cm³.

6. Micro-éponges d'acide hyaluronique selon la revendication 5, **caractérisées en ce qu'**elles ont un diamètre moyen situé dans la plage allant de 4,0 à 8,0 µm, une taille moyenne des pores située dans la plage allant de 100 à 300 nm, et un degré de réticulation situé dans la plage allant de 0,005 à 0,008 mol/cm³.

7. Micro-éponges d'acide hyaluronique selon la revendication 5 ou 6, **caractérisées en ce qu'**elles incorporent, à l'intérieur, des molécules, des macromolécules et des nanoparticules ayant une charge positive.

8. Micro-éponges d'acide hyaluronique selon l'une quelconque des revendications 5 à 7, pour utilisation dans des applications médicales ou cosmétiques.

9. Composé à usage médical ou cosmétique, **caractérisé en ce qu'**il comprend des micro-éponges d'acide hyaluronique selon l'une quelconque des revendications 5 à 7.
